# EUROPEAN PATENT APPLICATION

(11) **EP 4 434 999 A1**
(43) Date of publication of application: **25.09.2024**
(21) Application number: 22894958.2
(22) Date of filing: 18.11.2022
(51) Int. Cl.: C07K 14/705, C07K 19/00, C12N 15/62, A61K 47/68, A61K 38/17, A61P 35/00, A61P 37/02

(54) **SIRP? VARIANT AND USE THEREOF**

(30) Priority: 19.11.2021 CN 202111376099; 26.11.2021 CN 202111425620
(71) Applicant: Hangzhou Sumgen Biotech Co., Ltd., Hangzhou, Zhejiang 310056 (CN); Sumgen Mab (Beijing) Biotech Co., Ltd., Beijing 100176 (CN)
(72) Inventor: LV, Ming, Beijing 100176 (CN); DING, Xiaoran, Beijing 100176 (CN); MIAO, Shiwei, Beijing 100176 (CN); TAN, Bin, Beijing 100176 (CN)
(74) Representative: Weickmann & Weickmann PartmbB
(86) International application number: PCT/CN2022/132893
(87) International publication number: WO 2023/088429

(57) **Abstract**

Provided is a SIRPα variant and a fusion protein thereof, capable of specifically blocking interaction between CD47 protein and SIRPα, not causing coagulation reaction, and capable of inhibiting growth and/or proliferation of tumors or tumor cells.

## Description

### TECHNICAL FIELD

The present application relates to the field of biomedicine, in particular to an SIRPα variant and a fusion protein thereof, as well as use thereof.

### BACKGROUND

CD47 protein is a transmembrane glycoprotein, belonging to a member of the immunoglobulin superfamily, expressed on the surface of many cells including erythrocytes. Ligands of CD47 include integrins, thrombospondin-1, and Signal Regulatory Proteins (SIRPs). CD47 affects a variety of biological functions, including cell migration, T cell and dendritic cell activation, axon development, etc. In addition, CD47 can inhibit the phagocytosis of macrophages by interacting with SIRPα and protect normal cells such as blood cells from being phagocytized by macrophages. Studies have found that in addition to normal tissue cells expressing CD47, many tumor cells overexpress CD47 and prevent the macrophages from phagocytosis of tumor cells by binding to SIRPα on the surface of macrophages. This is regarded as a mechanism for tumors to evade the body's immune surveillance. Blocking the interaction between CD47 protein and SIRPα can inhibit tumor growth (Theocharides APA, *et al.,* 2012).

However, the existing reagents for blocking the interaction between CD47 protein and SIRPα have limited recognition activity, their affinity to CD47 protein is often insufficient, and their ability to inhibit tumors is limited. On the other hand, the existing antibody drugs targeting CD47 have side effects of causing anemia or thrombocytopenia (Bai Yinpeng et al., Chin J Clin Oncol., 2017 Vol 44. No.7). There is an urgent need to develop new therapies that can effectively block the interaction between CD47 protein and SIRPα and have few side effects.

### SUMMARY OF THE INVENTION

The present application provides an SIRPα variant and an SIRPα-Fc fusion protein, as well as use thereof. The fusion protein can specifically bind to CD47 protein. The SIRPα variants and fusion proteins thereof of the present application have one or more of the following properties: specifically binding to CD47 protein with high affinity; 2) specifically blocking the interaction between CD47 protein and SIRPα; 3) not inducing coagulation reaction; and 4) inhibiting the growth and/or proliferation of tumors or tumor cells. The present application further provides a preparation method and use of the SIRPα variant and a fusion protein thereof.

In one aspect, the present application provides an SIRPα variant, which, compared to a domain of the human SIRPα variant 1 or a fragment thereof, comprises an amino acid substitution at the amino acid residue of N110.

In certain embodiments, the domain of the human SIRPα variant 1 or fragment thereof comprises the amino acid residues at position of 33 -149 of the human SIRPα variant 1.

In certain embodiments, the amino acid residues at position of 33 -149 of the human SIRPα variant 1 comprise the amino acid sequence as set forth in SEQ ID NO: 2.

In certain embodiments, the SIRPα variant comprises an amino acid substitution of N110A.

In certain embodiments, the SIRPα variant further comprises an amino acid substitution at one or more residues selected from the group consisting of: L44, 161, V63, E77, Q82, K83, E84, V93, D95, L96, K98, N100, R107, G109 and V132.

In certain embodiments, the SIRPα variant further comprises one or more amino acid substitutions selected from the group consisting of: L44V, I61L/V/F, V63I, E77I/N/Q/K/H/M/R/V/L, Q82S/R/G/N, K83R, E84Q/K/H/D/R/G, V93L/A, D95H/R/E, L96S/T, K98R, N100G/K/D/E, R107N/S, G109R/H and V132L/R/I/S.

In certain embodiments, the SIRPα variant comprises substitutions at amino acid residues selected from any of the following groups:
(1) 161, V63, E77, E84, V93, L96, K98, N100, N110 and V132;
(2) 161, E77, Q82, K83, E84 and N110;
(3) I61, V63, K83, E84, N110 and V132;
(4) 161, E77, E84, R107, N110 and V132;
(5) 161, V63, E77, K83, E84, N100 and N110;
(6) I61, E77, Q82, K83, E84, R107 and N110;
(7) I61, E77, Q82, E84, V93, L96, N100, R107, G109, N110 and V132;
(8) I61, E77, Q82, K83, E84, N110 and V132;
(9) I61 and N110;
(10) 161, D95, L96, G109, N110 and V132;
(11) 161, D95, L96, K98, G109, N110 and V132;
(12) 161, E77, E84, V93, R107, N110 and V132;
(13) E77, L96, N100, G109, N110 and V132;
(14) 161, V63, Q82, E84, D95, L96, N100, N110 and V132;
(15) 161, E77, Q82, K83, E84, V93, D95, L96, K98, N100, N110 and V132;
(16) 161, E77, Q82, K83, E84, V93 and N110;
(17) 161, V63, E77, K83, E84, D95, L96, K98, N100 and N110;
(18) 161, V63, E77, K83, D95, L96, K98, N100, G109 and N110;
(19) 161, E77, Q82, E84, V93, D95, L96, K98, N100 and N110;
(20) 161, V63, E77, Q82, E84 and N110; and
(21) L44, 161, E77, Q82, K83, E84, N110 and V132.

In certain embodiments, the SIRPα variant comprises amino acid substitutions at residues of L44, 161, E77, Q82, K83, E84, N110, and V132.

In certain embodiments, the SIRPα variant comprises amino acid mutations selected from any of the following groups:
(1) I61L, V63I, E77I, E84K, V93L, L96S, K98R, N100G, N110A and V132L;
(2) 161V, E77N, Q82S, K83R, E84H and N110A;
(3) I61F, V63I, K83R, E84K, N110A and V132I;
(4) I61L, E77Q, E84D, R107N, N110A and V132I;
(5) I61L, V63I, E77K, K83R, E84D, N100G and N110A;
(6) 161V, E77H, Q82R, K83R, E84H, R107S and N110A;
(7) I61L, E77I, Q82G, E84R, V93L, L96T, N100G, R107S, G109R, N110A and V132R;
(8) I61L, E77M, Q82G, K83R, E84D, N110A and V132L;
(9) I61L and N110A;
(10) I61F, D95H, L96S, G109H, N110A and V132S;
(11) I61F, D95H, L96S, K98R, G109H, N110A and V132S;
(12) I61L, E77Q, E84D, V93A, R107N, N110A and V132I;
(13) E77K, L96S, N100K, G109H, N110A and V132L;
(14) I61L, V63I, Q82G, E84G, D95R, L96S, N100D, N110A and V132I;
(15) I61L, E77R, Q82N, K83R, E84G, V93L, D95E, L96T, K98R, N100D, N110A, and V132L;
(16) 161V, E77N, Q82S, K83R, E84H, V93A and N110A;
(17) 161V, V63I, E77V, K83R, E84D, D95E, L96T, K98R, N100E and N110A;
(18) 161L, V63I, E77V, K83R, D95E, L96S, K98R, N100D, G109R and N110A;
(19) I61V, E77L, Q82G, E84G, V93L, D95E, L96T, K98R, N100G and N110A;
(20) 161L, V63I, E77N, Q82G, E84G and N110A; and
(21) L44V, I61F, E77I, Q82R, K83R, E84Q, N110A and V132I.

In certain embodiments, the SIRPα variant comprises amino acid mutations of L44V, I61F, E77I, Q82R, K83R, E84Q, N110A, and V132I.

In certain embodiments, the SIRPα variant comprises an amino acid sequence as set forth in any one of SEQ ID NOs: 1, and 8-27.

In another aspect, the present application further provides a fusion protein comprising the SIRPα variant of the present application, and an immunoglobulin Fc region.

In certain embodiments, the immunoglobulin Fc region comprises the Fc region of IgG or a variant thereof.

In certain embodiments, the IgG is selected from the group consisting of IgG1, IgG2, IgG3, and IgG4.

In certain embodiments, the SIRPα variant in the fusion protein is located at the N-terminus of the immunoglobulin Fc region.

In certain embodiments, the immunoglobulin Fc region comprises an amino acid sequence selected from the group consisting of: SEQ ID NOs: 4-5.

In certain embodiments, the fusion protein comprises the amino acid sequence as set forth in any one of SEQ ID NOs: 6-7.

In certain embodiments, the fusion protein specifically binds to CD47 protein and has at least one of the following properties: 1) binding to CD47 protein with a *K_{D}* value of 1 × 10⁻⁸M or lower; 2) specifically blocking the interaction between CD47 protein and SIRPα; 3) not inducing coagulation reaction; and 4) inhibiting the growth and/or proliferation of tumors or tumor cells.

In certain embodiments, the CD47 protein is human CD47 protein.

In another aspect, the present application further provides one or more isolated nucleic acid molecules encoding the SIRPα variant described in the present application, or the fusion protein described in the present application.

In another aspect, the present application further provides a vector comprising the nucleic acid molecule described in the present application.

In another aspect, the present application further provides a host cell comprising the nucleic acid molecule or the vector.

In another aspect, the present application further provides a method for preparing the SIRPα variant or the fusion protein, the method comprising culturing the host cell under conditions that allow the expression of the SIRPα variant or the fusion protein.

In another aspect, the present application further provides a composition comprising the SIRPα variant, the fusion protein, the nucleic acid molecule, the vector and/or the host cell, and optionally a pharmaceutically acceptable adjuvant.

In another aspect, the present application further provides use of the SIRPα variant, the fusion protein, the nucleic acid molecule, the vector, the host cell and/or the composition in the preparation of drugs and/or kits for preventing or treating tumors or autoimmune diseases.

In certain embodiments, the tumor is selected from the group consisting of CD47-positive hematological tumors or CD47-positive solid tumors.

In certain embodiments, the autoimmune disease is selected from the group consisting of Crohn's disease, allergic asthma, and rheumatoid arthritis.

In another aspect, the present application further provides a method for blocking the interaction between CD47 protein and SIRPα, which comprises administering the fusion protein or the composition. In certain embodiments, the method is an in vitro method. In certain embodiments, the method is an ex vivo method.

Those skilled in the art will readily appreciate other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
Fig. 1 shows a schematic diagram of the physical structure of vector pTM.
Fig. 2 shows a schematic diagram of a method for detecting the interaction between SIRPα truncated domains and mutants thereof and CD47.
Fig. 3 shows the results of screening by flow cytometry of SIRPα truncated domains.
Fig. 4 shows the results of recognition of the CD47 protein by the fusion protein of the present application.
Fig. 5 shows the results of detection for affinity between the fusion protein of the present application and CD47.
Fig. 6 shows the results of blocking the binding of CD47 to SIRPα by the fusion protein of the present application .
Figs. 7A-7D show the results of binding of the fusion protein of the present application to Raji cells and A549 cells.
Fig. 8 shows the results of detection for binding of the fusion protein of the present application to human erythrocytes.
Fig. 9 shows the anti-hemagglutination effect of the fusion protein of the present application.
Fig. 10 shows the detection for in vivo tumor inhibitory effect of the fusion protein of the present application.

### DETAILED DESCRIPTION OF THE INVENTION

The embodiments of the invention of the present application will be described below with specific examples. Those skilled in the art can easily understand other advantages and effects of the invention of the present application from the disclosure of the specification.

### Definition of Terms

In the present application, the term "CD47 protein", also known as integrin associated protein (IAP), belongs to the immunoglobulin superfamily, and may bind to membrane integrins and their ligands, i.e., thrombospondin-1 (TSP-1), and signal-regulatory protein alpha (SIRPα). CD47 protein is widely expressed on the surface of cell membranes and is a supramolecular complex composed of specific integrins, G proteins and cholesterol. In the present application, the CD47 protein may be human CD47 protein. For example, the CD47 protein may be CD47 protein expressed on a cell surface.

In the present application, the term "CD47 positive" generally refers to the characteristic of expressing CD47 protein or fragment thereof on the surface of an organism or cell. In particular, the "CD47 positive" cells described in the present application may be those cells that overexpress CD47. These CD47 positive cells are typically disease cells, and the CD47 protein density on their surface exceeds the normal CD47 protein density possessed by the specified cell type. In certain embodiments, the tumor or tumor cells may be CD47 positive. For example, the tumor may be selected from the group consisting of CD47-positive hematological tumors or CD47-positive solid tumors.

In the present application, the term "*K_{D}*" may be used interchangeably with "KD", and generally refers to the dissociation equilibrium constant of a particular antibody-antigen interaction, in M (mol/L). KD can be calculated from the concentrations of substance AB and substance A and substance B resulting from its dissociation: KD =c(A)*c(B)/c(AB). As known from this formula, the larger the KD value, the more dissociation there is, which means the weaker the affinity between substances A and B; conversely, the smaller the KD value, the less dissociation there is, which means the stronger the affinity between substances A and B.

In the present application, the term "SIRPα" generally refers to a regulatory membrane glycoprotein from the SIRP family, which may act as a ligand for the CD47 protein. SIRPα is a transmembrane protein whose extracellular region contains 3 immunoglobulin superfamily-like regions, of which the N-terminal region mediates binding to CD47. It is mainly expressed on the surface of macrophages, dendritic cells and nerve cells.

In the present application, the term "human SIRPα domain" generally refers to the wild-type, endogenous mature form of human SIRPα, a fragment or functional variant thereof. In humans, the SIRPα protein is found to have two main forms. The amino acid sequence of one form (variant 1 or type V1) is listed as NCBI RefSeq NP_542970.1 (residues 31-504 constitute the mature form). Another form (variant 2 or type V2) differs from the variant 1 or type V1 by 13 amino acids and its amino acid sequence is listed in GenBank as CAA71403.1. These two forms of SIRPα constitute approximately 80% of the various types of SIRPα present in humans.

In the present application, the term "immunoglobulin Fc region" generally refers to the base region of the Y-shaped structure of the antibody structure, also known as the Fragment crystallizable region (Fc region). In the IgG, IgA, and IgD antibody isotypes, the Fc region consists of two identical protein fragments derived from the second and third constant domains of the two heavy chains of the antibody; and the Fc regions of IgM and IgE contain three heavy chain constant domains in each polypeptide chain. The Fc region of IgG has a highly conserved N-glycosylation site. In certain embodiments, the immunoglobulin Fc region may comprise the Fc region of IgG. For example, the immunoglobulin Fc region may comprise an amino acid sequence selected from the group consisting of: SEQ ID NOs: 4-5, or a functional variant thereof.

In the present application, the term "IgG" generally refers to immunoglobulin G. IgG is one of the human immunoglobulins, and the others are IgA, IgM, IgD and IgE. According to the antigenic differences of the gamma chain in IgG molecules, there are four subtypes for human IgG: IgG1, IgG2, IgG3 and IgG4. In the present application, the term "IgG1" generally refers to a subtype with the highest proportion in IgG, which has a higher affinity for the Fc receptor. For example, the IgG may be human IgG. As another example, the IgG may be selected from the group consisting of IgG1 and IgG4.

In the present application, the human SIRPα domain may be located at the N-terminus of the immunoglobulin Fc region. For example, the human SIRPα domain and the immunoglobulin Fc can be linked via a linker. For example, in the present application, the human SIRPα domain may comprise the amino acid sequence as set forth in SEQ ID NO: 1.

In the present application, the human SIRPα domain may comprise the domain of the human SIRPα variant 1, and a fragment or variant thereof, the sequence of which may comprise the amino acid sequence as set forth in SEQ ID NO: 2 or a functional variant thereof. For example, the human SIRPα domain may comprise the IgV domain of the human SIRPα variant 1, and a fragment or variant thereof, the sequence of which may comprise the residues at position 38-145 in the amino acid sequence as set forth in SEQ ID NO: 3 or a functional variant thereof. As another example, the human SIRPα domain may comprise a truncated domain of the human SIRPα variant 1, which may comprise the amino acid sequence as set forth in SEQ ID NO: 2 (i.e., the residues at position 33-149 in the amino acid sequence as set forth in SEQ ID NO: 3) or a functional variant thereof. The human SIRPα domain of the present application may comprise an extracellular domain of the human SIRPα, and a fragment or variant thereof. For example, the human SIRPα domain may comprise the IgV domain of human SIRPα, and a fragment or variant thereof.

In the present application, the fusion protein may include a human SIRPα domain capable of specifically binding to the CD47 protein and an immunoglobulin Fc region, wherein the human SIRPα domain is directly or indirectly linked to the immunoglobulin Fc region.

The variants of the SIRPα domain described in the present application may comprise an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the amino acid sequence as set forth in SEQ ID NO: 1.

The variants of the fusion protein described in the present application may comprise an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the amino acid sequence as set forth in any one of SEQ ID NOs: 6-7.

In the present application, the term "sequence homology" generally refers to sequence similarity or interchangeability between two or more polynucleotide sequences or between two or more polypeptide sequences. When using a computer program or software (e.g., Emboss Needle or BestFit) to determine sequence identity, similarity, or homology between different amino acid sequences, default parameter settings may be used. An appropriate scoring matrix, such as blosum45 or blosum80, can also be selected to optimize identity, similarity or homology scores. In certain embodiments, homologous polynucleotides include polynucleotides that are capable of hybridizing to a control polynucleotide sequence under stringent conditions and have at least 60%, at least 65%, at least 70%, at least 80%, at least 90%, at least 95%, at least 97%, at least 98%, at least 99% or even at least 100% sequence identity compared with the control polynucleotide sequence. A homologous polypeptide may be a polypeptide that has at least 80%, at least 85%, at least 90%, at least 95%, at least 97%, at least 98%, at least 99%, or even at least 100% sequence identity to the control polypeptide sequence when the sequences are aligned under optimized conditions.

To determine sequence identity, sequence alignment can be performed, which can be performed by various means known to those skilled in the art, for example, using BLAST, BLAST-2, ALIGN, NEEDLE or Megalign (DNASTAR) software, etc. One skilled in the art will be able to determine appropriate parameters for alignment, including any algorithms required to achieve optimal alignment across the full-length sequences being compared.

In the present application, a variant of an amino acid sequence (e.g., a protein domain or a protein fragment, such as the human SIRPα domain described in the present application, or the fusion protein described in the present application) may comprise the substitution, deletion, or addition of one or more amino acid residues. In the present application, the variant (e.g., a variant of the human SIRPα domain) may comprise an amino acid substitution at one or more residues selected from the group consisting of: L44, 161, E77, Q82, K83, E84, N110 and V132.

In the present application, the position of the amino acid residue in the amino acid substitution is numbered by the residue determined based on the amino acid sequence as set forth in SEQ ID NO: 3.

In the present application, "residue Xn" refers to residue X corresponding to the nth position of the amino acid sequence as set forth in SEQ ID NO: 3, where n is a positive integer and X is an abbreviation for any amino acid residue. For example, "residue 161" represents the amino acid residue I corresponding to the 61st position of the amino acid sequence as set forth in SEQ ID NO: 3.

In the present application, according to the above numbering scheme, the number of the first amino acid of the amino acid sequence as set forth in SEQ ID NO: 2 is the amino acid at position 33. Therefore, "residue 161" represents the amino acid residue I corresponding to the 29th position of the amino acid sequence as set forth in SEQ ID NO: 2. That is, the number of the amino acid at position 29 of the amino acid sequence as set forth in SEQ ID NO: 2 is the amino acid at position 61.

In the present application, "amino acid substitution Xn" means that an amino acid substitution occurs at residue X corresponding to the nth position of amino acid sequence as set forth in SEQ ID NO: 3, where n is a positive integer and X is an abbreviation for any amino acid residue. For example, "amino acid substitution 161" represents that amino acid substitution occurs at residue I corresponding to the 61st position of the amino acid sequence as set forth in SEQ ID NO: 3, and also represents that amino acid substitution occurs a residue corresponding to the 29th amino acid residue of the amino acid sequence as set forth in SEQ ID NO: 2.

In the present application, a certain residue in a certain amino acid sequence "corresponding to" a certain residue in another amino acid sequence generally refers to the residue correspondence obtained when the amino acid sequence alignment is performed under optimized conditions. The sequence alignment can be performed in a manner understood by those skilled in the art, for example, using BLAST, BLAST-2, ALIGN, NEEDLE or Megalign (DNASTAR) software. One skilled in the art will be able to determine appropriate parameters for alignment, including any algorithms required to achieve optimal alignment across the full-length sequences being compared.

The amino acid substitutions described in the present application may be non-conservative substitutions. The non-conservative substitution may include changing the amino acid residues in the target protein or polypeptide in a non-conservative manner, for example, changing an amino acid residue with a certain side chain size or a certain property (for example, hydrophilicity) to an amino acid residue with a different side chain size or different property (e.g., hydrophobicity).

The amino acid substitutions may also be conservative substitutions. The conservative substitution may include changing the amino acid residues in the target protein or polypeptide in a conservative manner, for example, changing an amino acid residue with a certain side chain size or a certain property (for example, hydrophilicity) to an amino acid residue with an identical or similar side chain size or identical or similar property (e.g., still hydrophilicity). Such conservative substitutions generally do not have a significant impact on the structure or function of the resulting protein. In the present application, amino acid sequence variants as the fusion proteins or fragments thereof may include conservative amino acid substitutions that do not significantly alter the structure of the protein or its function (e.g., the ability to block the binding of CD47 to its ligand).

As an example, the mutual substitutions between the amino acids in each group in the following groups can be considered as conservative substitutions in the present application:
Group of amino acids with nonpolar side chains: alanine, valine, leucine, isoleucine, proline, phenylalanine, tryptophan, and methionine.
Group of uncharged amino acids with polar side chains: glycine, serine, threonine, cysteine, tyrosine, asparagine, and glutamine.
Group of negatively charged amino acids with polar side chains: aspartic acid and glutamic acid.
Positively charged basic amino acids: lysine, arginine, and histidine.
Amino acids with phenyl: phenylalanine, tryptophan, and tyrosine.

In the present application, the amino acid substitution "XnY/Z" means that according to the numbering scheme described in the present application, the residue X at position n in the amino acid sequence is substituted with the amino acid residue Y or the amino acid residue Z, wherein n is a positive integer, and X, Y and Z are independently abbreviated for any amino acid residue respectively, and X is different from Y or Z. For example, the amino acid substitution "I61L/V/F" refers to that the residue I at position 61 is substituted with the amino acid residues L, V or F.

### Detailed Description of the Invention

### SIRPα variants

In one aspect, the present application provides an SIRPα variant, which compared with the domain of the human SIRPα variant 1 or a fragment thereof, comprises an amino acid substitution at the amino acid residue of N110.

In certain embodiments, the domain of the human SIRPα variant 1 may comprise the extracellular domain of the human SIRPα variant 1. In certain embodiments, the domain of the human SIRPα variant 1 may comprise the IgV domain of the human SIRPα variant 1. In certain embodiments, the domain of the human SIRPα variant 1 or fragment thereof may comprise a truncated domain of the human SIRPα variant 1.

In certain embodiments, the truncated domain of the human SIRPα variant 1 may have the residues at position 33-149 in the amino acid sequence as set forth in SEQ ID NO: 3. For example, the truncated domain of the human SIRPα variant 1 may comprise the amino acid sequence as set forth in SEQ ID NO: 2.

In certain embodiments, the SIRPα variant may comprise the amino acid substitution N110A compared with the amino acid sequence as set forth in SEQ ID NO: 2.

In certain embodiments, compared with the domain of the human SIRPα variant 1 or fragment thereof, the SIRPα variant comprises an amino acid substitution at the amino acid residue of L44.

In certain embodiments, the SIRPα variant may comprise the amino acid substitution L44V compared with the amino acid sequence as set forth in SEQ ID NO: 2.

In certain embodiments, compared with the amino acid sequence as set forth in SEQ ID NO: 2, the SIRPα variant may comprise amino acid substitutions at one or more (e.g., 1-2, 1-3, 1-4, 1-5, 1-6, 1-7, 1-8, 1-9, 1-10 or more) amino acid residues selected from the group consisting of L44, I61, V63, E77, Q82, K83, E84, V93, D95, L96, K98, N100, R107, G109, N110 and V132. Among them, the numbering of the amino acid sites is confirmed based on the amino acid sequence as set forth in SEQ ID NO: 3.

In certain embodiments, compared with the amino acid sequence as set forth in SEQ ID NO: 2, the SIRPα variant may comprise substitutions at amino acid positions selected from any of the following groups: (1) I61, V63, E77, E84, V93, L96, K98, N100, N110 and V132; (2) I61, E77, Q82, K83, E84 and N110; (3) I61, V63, K83, E84, N110 and V132; (4) I61, E77, E84, R107, N110 and V132; (5) I61, V63, E77, K83, E84, N100 and N110; (6) I61, E77, Q82, K83, E84, R107 and N110; (7) I61, E77, Q82, E84, V93, L96, N100, R107, G109, N110 and V132; (8) I61, E77, Q82, K83, E84, N110 and V132; (9) I61 and N110; (10) I61, D95, L96, G109, N110 and V132; (11) I61, D95, L96, K98, G109, N110 and V132; (12) I61, E77, E84, V93, R107, N110 and V132; (13) E77, L96, N100, G109, N110 and V132; (14) I61, V63, Q82, E84, D95, L96, N100, N110 and V132; (15) I61, E77, Q82, K83, E84, V93, D95, L96, K98, N100, N110 and V132; (16) I61, E77, Q82, K83, E84, V93 and N110; (17) 161, V63, E77, K83, E84, D95, L96, K98, N100 and N110; (18) I61, V63, E77, K83, D95, L96, K98, N100, G109 and N110; (19) I61, E77, Q82, E84, V93, D95, L96, K98, N100 and N110; (20) I61, V63, E77, Q82, E84 and N110; and (21) L44, 161, E77, Q82, K83, E84, N110 and V132. Among them, the numbering of the amino acid sites is confirmed based on the amino acid sequence as set forth in SEQ ID NO: 3. In certain embodiments, compared with the amino acid sequence as set forth in SEQ ID NO: 2, the mutant may comprise one or more (e.g., 1-2, 1-3, 1-4, 1-5, 1-6, 1-7, 1-8, 1-9, 1-10 or more) amino acid substitutions selected from the group consisting of L44V, I61L/V/F, V63I, E77I/N/Q/K/H/M/R/V/L, Q82S/R/G/N, K83R, E84Q/K/H/D/R/G, V93L/A, D95H/R/E, L96S/T, K98R, N100G/K/D/E, R107N/S, G109R/H, N110A and V132L/R/I/S. Among them, the numbering of the amino acid sites is confirmed based on the amino acid sequence as set forth in SEQ ID NO: 3.

For example, in the present application, compared with the amino acid sequence as set forth in SEQ ID NO: 2, the SIRPα variant may also comprise mutations at one or more other sites in addition to the N110A mutation. For example, the SIRPα variant may comprise L44V amino acid mutation. For example, the SIRPα variant may comprise I61L, I61V or I61F amino acid mutation. For example, the SIRPα variant may comprise V63I amino acid mutation. For example, the SIRPα variant may comprise E77I, E77N, E77Q, E77K, E77H, E77M, E77R, E77V or E77L amino acid mutation. For example, the SIRPα variant may comprise Q82S, Q82R, Q82G or Q82N amino acid mutation. For example, the SIRPα variant may comprise K83R amino acid mutation. For example, the SIRPα variant may comprise E84Q, E84K, E84H, E84D, E84R or E84G amino acid mutation. For example, the SIRPα variant may comprise V93L or V93A amino acid mutation. For example, the SIRPα variant may comprise D95H, D95R or D95E amino acid mutation. For example, the SIRPα variant may comprise L96S or L96T amino acid mutation. For example, the SIRPα variant may comprise K98R amino acid mutation. For example, the SIRPα variant may comprise N100G, N100K, N100D or N100E amino acid mutation. For example, the SIRPα variant may comprise R107N or R107S amino acid mutation. For example, the SIRPα variant may comprise G109R or G109H amino acid mutation. For example, the SIRPα variant may comprise V132L, V132R, V132I or V132S amino acid mutation.

In the present application, the SIRPα variant may comprise substitutions at the following amino acid positions: I61, E77 and N110. For example, the SIRPα variant may comprise the amino acid substitutions of I61L/V/F, E77Q/N/I, and N110A.

In the present application, the SIRPα variant may comprise substitutions at the following amino acid positions: I61, N110 and V132. For example, the SIRPα variant may comprise the amino acid substitutions of I61L/V/F, N110A and V132I/S.

In the present application, the SIRPα variant may comprise substitutions at the following amino acid positions: I61, E84 and N110. For example, the SIRPα variant may comprise the amino acid substitutions of I61L/V/F, E84D/H, and N110A.

In the present application, the SIRPα variant may comprise substitutions at the following amino acid positions: I61, K83, E84 and N110. For example, the SIRPα variant may comprise the amino acid substitutions of I61L/V/F, K83R, E84Q/D/H and N110A.

In the present application, the SIRPα variant may comprise substitutions at the following amino acid positions: I61, D95, L96 and N110. For example, the SIRPα variant may comprise the amino acid substitutions of I61L/V/F, D95H, L96S and N110.

In the present application, the SIRPα variant may comprise substitutions at the following amino acid positions: I61, D95, L96, G109, N110 and V132. For example, the SIRPα variant may comprise the amino acid substitutions of I61L/V/F, D95H, L96S, G109H, N110A and V132I/S.

In the present application, the SIRPα variant may comprise substitutions at the following amino acid positions: I61, G109, N110 and V132. For example, the SIRPα variant may comprise the amino acid substitutions of I61L/V/F, G109H, N110A and V132I/S.

In the present application, the SIRPα variant may comprise substitutions at the following amino acid positions: V93, R107 and N110. For example, the SIRPα variant may comprise the amino acid substitutions of V93A, R107N and N110A.

In the present application, the SIRPα variant may comprise substitutions at the following amino acid positions: L44, I61 and N110. For example, the SIRPα variant may comprise the amino acid substitutions of L44V, I61L/V/F and N110A.

In the present application, the SIRPα variant may comprise substitutions at the following amino acid positions: L44, E77 and N110. For example, the SIRPα variant may comprise the amino acid substitutions of L44V, E77Q/N/I and N110.

In the present application, the SIRPα variant may comprise substitutions at the following amino acid positions: Q82, K83, E84 and N110. For example, the SIRPα variant may comprise the amino acid substitutions of Q82S/R/G/N, K83R, E84Q/K/H/D/R/G and N110A.

In the present application, the SIRPα variant may comprise substitutions at the following amino acid positions: E77, N110 and V132. For example, the SIRPα variant may comprise the amino acid substitutions of E77Q/N/I, N110A and V132I/S.

In certain embodiments, compared to the amino acid sequence as set forth in SEQ ID NO: 2, the SIRPα variant may further comprise an amino acid substitution at one or more residues selected from the group consisting of: L44, I61, E77, Q82, K83, E84, N110 and V132. In certain embodiments, the amino acid substitution can be selected from: L44V, I61L/V/F, E77I/N/Q/K/H/M/R/N/V/L, Q82S/R/G/N, K83R, E84Q/K/H/D/R/G and V132L/R/I/S. In the present application, the numbering of the amino acid sites is confirmed based on SEQ ID NO: 3.

In certain embodiments, the SIRPα variant may comprise amino acid substitutions selected from any of the following groups: (1) I61L, V63I, E77I, E84K, V93L, L96S, K98R, N100G, N110A and V132L; (2) 161V, E77N, Q82S, K83R, E84H and N110A; (3) I61F, V63I, K83R, E84K, N110A and V132I; (4) I61L, E77Q, E84D, R107N, N110A and V132I; (5) I61L, V63I, E77K, K83R, E84D, N100G and N110A; (6) 161V, E77H, Q82R, K83R, E84H, R107S and N110A; (7) I61L, E77I, Q82G, E84R, V93L, L96T, N100G, R107S, G109R, N110A and V132R; (8) I61L, E77M, Q82G, K83R, E84D, N110A and V132L; (9) I61L and N110A; (10) I61F, D95H, L96S, G109H, N110A and V132S; (11) I61F, D95H, L96S, K98R, G109H, N110A and V132S; (12) I61L, E77Q, E84D, V93A, R107N, N110A and V132I; (13) E77K, L96S, N100K, G109H, N110A and V132L; (14) I61L, V63I, Q82G, E84G, D95R, L96S, N100D, N110A and V132I; (15) I61L, E77R, Q82N, K83R, E84G, V93L, D95E, L96T, K98R, N100D, N110A and V132L; (16) 161V, E77N, Q82S, K83R, E84H, V93A and N110A; (17) 161V, V63I, E77V, K83R, E84D, D95E, L96T, K98R, N100E and N110A; (18) I61L, V63I, E77V, K83R, D95E, L96S, K98R, N100D, G109R and N110A; (19) 161V, E77L, Q82G, E84G, V93L, D95E, L96T, K98R, N100G and N110A; (20) I61L, V63I, E77N, Q82G, E84G and N110A; and (21) L44V, I61F, E77I, Q82R, K83R, E84Q, N110A and V132I.

In certain embodiments, compared to the amino acid sequence as set forth in SEQ ID NO: 2, the SIRPα variant may comprise amino acid substitutions at amino acid residues of L44, I61, E77, Q82, K83, E84, N110 and V132. In certain embodiments, compared to the amino acid sequence as set forth in SEQ ID NO: 2, the SIRPα variant may comprise amino acid mutations of L44V, I61F, E77I, Q82R, K83R, E84Q, N110A and V132I.

In certain embodiments, the SIRPα variant may comprise an amino acid sequence as set forth in SEQ ID NO: 1.

In the present application, on the basis of the truncated domain of the human SIRPα variant 1 (the amino acid sequence as set forth in SEQ ID NO: 2, that is, residues at position 33-149 in the amino acid sequence of the human SIRPα variant 1), the mutants of SIRPα variant 1 comprising the above amino acid substitution groups (1) to (21) respectively can be named m1N, m5N, m170N, m35N, m37N, m41N, m57N, m67N, m81N, m82N, m84N, m91N, m99N, m102N, m111N, m122N, m126N, m130N, m135N, m145N and m12N in turn. The mutants of SIRPα variant 1 may in turn comprise the amino acid sequence as set forth in any one of SEQ ID NOs: 1, and 8-27. In the present application, the mutant of the SIRPα variant 1 may comprise an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology to the amino acid sequence as set forth in any one of SEQ ID NOs: 1, and 8-27.

### Fusion protein

In another aspect, the present application provides a fusion protein comprising the SIRPα variant described in the present application. In certain embodiments, the fusion protein may specifically bind to the CD47 protein, and bind to the CD47 protein with a *K_{D}* value of 1 × 10⁻⁸M or less, for example: the *K_{D}* value is no greater than 9×10⁻⁹M, no greater than 8×10⁻⁹M, no greater than 7×10⁻⁹M, no greater than 6.2×10⁻⁹M, no greater than 6×10⁻⁹M, no greater than 5×10⁻⁹M, no greater than 4.8×10⁻⁹M, no greater than 4.5×10⁻⁹M, no greater than 2×10⁻⁹M, no greater than 1.5 × 10⁻⁹M, no greater than 1×10⁻⁹M or no greater than 1×10⁻¹⁰M or less.

In certain embodiments, the fusion protein can specifically block the interaction between CD47 protein and SIRPα, thereby activating macrophages to phagocytose tumor cells. In addition, the fusion protein described in the present application may not cause coagulation reaction, for example, by test with a blood agglutination plate, to which the fusion protein and an erythrocyte solution are added, then the erythrocytes settle at the bottom of the well without spreading out in a network shape. The fusion protein can also inhibit the growth and/or proliferation of tumors or tumor cells, for example, can reduce tumor area or tumor volume, or can improve the survival of subjects bearing tumors.

In the present application, the term "fusion protein" generally refers to a composite polypeptide, i.e., a single continuous amino acid sequence consisting of two (or more) polypeptides. Fusion proteins can generally be artificially prepared using nucleic acid recombination or chemical synthesis.

In the present application, the fusion protein may comprise an SIRPα variant, and an immunoglobulin Fc region. In certain embodiments, the immunoglobulin Fc region comprises the Fc region of IgG or a variant thereof. For example, the IgG may be selected from IgG1, IgG2, IgG3 and IgG4.

For example, the fusion protein may comprise an SIRPα variant, and an immunoglobulin Fc region, wherein compared with the amino acid sequence as set forth in SEQ ID NO: 2, the SIRPα variant comprises amino acid substitutions at residues of L44, I61, E77, Q82, K83, E84, N110 and V132.

For example, the fusion protein may comprise an SIRPα variant, and an immunoglobulin Fc region, wherein compared with the amino acid sequence as set forth in SEQ ID NO: 2, the SIRPα variant comprises amino acid mutations of L44V, I61F, E77I, Q82R, K83R, E84Q, N110A and V132I.

For example, the Fc region ofIgG 1 may comprise the amino acid sequence as set forth in SEQ ID NO: 4. For example, the Fc region of IgG4 may comprise the amino acid sequence as set forth in SEQ ID NO: 5.

In the present application, the fusion protein may comprise the SIRPα variant sequence as set forth in SEQ ID NO: 1, and the Fc region sequence as set forth in SEQ ID NO: 4 or SEQ ID NO: 5.

In the present application, the fusion protein may comprise the SIRPα variant sequence as set forth in SEQ ID NO: 8, and the Fc region sequence as set forth in SEQ ID NO: 4 or SEQ ID NO: 5.

In the present application, the fusion protein may comprise the SIRPα variant sequence as set forth in SEQ ID NO: 9, and the Fc region sequence as set forth in SEQ ID NO: 4 or SEQ ID NO: 5.

In the present application, the fusion protein may comprise the SIRPα variant sequence as set forth in SEQ ID NO: 10, and the Fc region sequence as set forth in SEQ ID NO: 4 or SEQ ID NO: 5.

In the present application, the fusion protein may comprise the SIRPα variant sequence as set forth in SEQ ID NO: 11, and the Fc region sequence as set forth in SEQ ID NO: 4 or SEQ ID NO: 5.

In the present application, the fusion protein may comprise the SIRPα variant sequence as set forth in SEQ ID NO: 12, and the Fc region sequence as set forth in SEQ ID NO: 4 or SEQ ID NO: 5.

In the present application, the fusion protein may comprise the SIRPα variant sequence as set forth in SEQ ID NO: 13, and the Fc region sequence as set forth in SEQ ID NO: 4 or SEQ ID NO: 5.

In the present application, the fusion protein may comprise the SIRPα variant sequence as set forth in SEQ ID NO: 14, and the Fc region sequence as set forth in SEQ ID NO: 4 or SEQ ID NO: 5.

In the present application, the fusion protein may comprise the SIRPα variant sequence as set forth in SEQ ID NO: 15, and the Fc region sequence as set forth in SEQ ID NO: 4 or SEQ ID NO: 5.

In the present application, the fusion protein may comprise the SIRPα variant sequence as set forth in SEQ ID NO: 16, and the Fc region sequence as set forth in SEQ ID NO: 4 or SEQ ID NO: 5.

In the present application, the fusion protein may comprise the SIRPα variant sequence as set forth in SEQ ID NO: 17, and the Fc region sequence as set forth in SEQ ID NO: 4 or SEQ ID NO: 5.

In the present application, the fusion protein may comprise the SIRPα variant sequence as set forth in SEQ ID NO: 18, and the Fc region sequence as set forth in SEQ ID NO: 4 or SEQ ID NO: 5.

In the present application, the fusion protein may comprise the SIRPα variant sequence as set forth in SEQ ID NO: 19, and the Fc region sequence as set forth in SEQ ID NO: 4 or SEQ ID NO: 5.

In the present application, the fusion protein may comprise the SIRPα variant sequence as set forth in SEQ ID NO: 20, and the Fc region sequence as set forth in SEQ ID NO: 4 or SEQ ID NO: 5.

In the present application, the fusion protein may comprise the SIRPα variant sequence as set forth in SEQ ID NO: 21, and the Fc region sequence as set forth in SEQ ID NO: 4 or SEQ ID NO: 5.

In the present application, the fusion protein may comprise the SIRPα variant sequence as set forth in SEQ ID NO: 22, and the Fc region sequence as set forth in SEQ ID NO: 4 or SEQ ID NO: 5.

In the present application, the fusion protein may comprise the SIRPα variant sequence as set forth in SEQ ID NO: 23, and the Fc region sequence as set forth in SEQ ID NO: 4 or SEQ ID NO: 5.

In the present application, the fusion protein may comprise the SIRPα variant sequence as set forth in SEQ ID NO: 24, and the Fc region sequence as set forth in SEQ ID NO: 4 or SEQ ID NO: 5.

In the present application, the fusion protein may comprise the SIRPα variant sequence as set forth in SEQ ID NO: 25, and the Fc region sequence as set forth in SEQ ID NO: 4 or SEQ ID NO: 5.

In the present application, the fusion protein may comprise the SIRPα variant sequence as set forth in SEQ ID NO: 26, and the Fc region sequence as set forth in SEQ ID NO: 4 or SEQ ID NO: 5.

In the present application, the fusion protein may comprise the SIRPα variant sequence as set forth in SEQ ID NO: 27, and the Fc region sequence as set forth in SEQ ID NO: 4 or SEQ ID NO: 5.

In the present application, the fusion protein may comprise an amino acid sequence as set forth in any one of SEQ ID NOs: 6-7.

In the present application, the fusion protein can specifically bind to CD47 protein and has at least one of the following properties: 1) binding to CD47 protein with *a K_{D}* value of 1×10⁻⁸M or lower; 2) specifically blocking the interaction between CD47 protein and SIRPα; 3) not inducing coagulation reaction; and 4) inhibiting the growth and/or proliferation of tumors or tumor cells.

In certain embodiments, the CD47 protein can be human CD47 protein.

### Nucleic acid molecules, vectors, and host cells

In another aspect, the present application provides one or more isolated nucleic acid molecules which can encode the SIRPα variant described in the present application or the fusion protein described in the present application.

In another aspect, the present application provides one or more vectors, which may comprise one or more nucleic acid molecules described in the present application. In another aspect, the present application provides a cell (e.g., a host cell) that may comprise the nucleic acid molecule described in the present application or the vector described in the present application.

In the present application, the term "nucleic acid molecule" generally refers to isolated forms of nucleotides, deoxyribonucleotides or ribonucleotides or their analogs of any length isolated from their natural environment or artificially synthesized. The nucleic acid molecules of the present application may be isolated. For example, it may be produced or synthesized by the following methods: (i) amplification in vitro, such as amplification by polymerase chain reaction (PCR), (ii) clonal recombination, (iii) purification, for example by enzymatic digestion and gel electrophoresis fractionation, or (iv) synthesis, for example, chemical synthesis. In certain embodiments, the isolated nucleic acid is a nucleic acid molecule prepared by recombinant DNA technology. In the present application, nucleic acids encoding the antibodies or antigen-binding fragments thereof can be prepared by a variety of methods known in the art, including but not limited to, overlap extension PCR using restricted fragment manipulation or using synthetic oligonucleotides. For specific operations, see Sambrook *et al.,* Molecular Cloning, A Laboratory Manual, Cold Spring Harbor Laboratory Press, Cold Spring Harbor, NY, 1989; and Ausube *et al.* Current Protocols in Molecular Biology, Greene Publishing and Wiley-Interscience, New York NY, 1993.

In the present application, the term "vector" generally refers to a nucleic acid molecule that is capable of self-replicating in a suitable host, transferring the inserted nucleic acid molecule to and/or between host cells. The vectors may include vectors primarily used for insertion of DNA or RNA into cells, vectors primarily used for replication of DNA or RNA, and vectors primarily used for expression of transcription and/or translation of DNA or RNA. The vectors also include vectors having a variety of the above-mentioned functions. The vector may be a polynucleotide capable of being transcribed and translated into a polypeptide when introduced into a suitable host cell. Generally, the vector can produce the desired expression product by culturing appropriate host cells comprising the vector. In the present application, one or more of the nucleic acid molecules may be comprised in the vector. In addition, the vector may further comprise other genes, such as a marker gene which allows the vector to be selected in a suitable host cell and under suitable conditions. In addition, the vector may further contain expression control elements that allow the coding region to be correctly expressed in an appropriate host. Such control elements are well known to those skilled in the art, and may include, for example, promoters, ribosome binding sites, enhancers, and other control elements that regulate gene transcription or mRNA translation. In certain embodiments, the expression control sequences are tunable elements. The specific structure of the expression control sequence can vary depending on the function of the species or cell type, but generally includes 5' non-transcribed sequences and 5' and 3' non-translated sequences involved in the initiation of transcription and translation, respectively, such as TATA box, capped sequence, and CAAT sequence. For example, the 5' non-transcribed expression control sequence may comprise a promoter region, which may comprise a promoter sequence for transcriptional control of the functionally linked nucleic acid. In the present application, the vector may be a pTM vector.

In the present application, the term "host cell", "cell", and "host" may be used interchangeably, and generally refer to individual cells, cell lines or cell cultures which may contain or have contained plasmid or vectors comprising nucleic acid molecules described in the present application, or which are capable of expressing fusion proteins, fragments or variants thereof described in the present application. The host cells may include progeny of a single host cell. Due to natural, accidental or intentional mutations, the progeny cells may not necessarily be completely identical in morphology or genome to the original parent cells, but they can express the antibodies or antigen-binding fragments thereof described in the present application. The host cells can be obtained by transfecting cells in vitro using the vectors of the present application. The host cells can be prokaryotic cells (such as E. coli) or eukaryotic cells (such as yeast cells, such as COS cells, Chinese hamster ovary (CHO) cells, HeLa cells, HEK293 cells, COS-1 cells, NS0 cells or myeloma cells). In the present application, the host cells may be CHO cells.

### Compositions, preparation methods and applications

In another aspect, the present application may provide a method for preparing the SIRPα variant or the fusion protein, which method may comprise culturing host cells under conditions that allow the expression of the fusion protein.

In another aspect, the present application may provide a composition, which may comprise the SIRPα variant, the fusion protein, the nucleic acid molecule, the vector and/or the host cell, and optionally a pharmaceutically acceptable adjuvant.

In the present application, the term "pharmaceutically acceptable adjuvants" may include buffers, antioxidants, preservatives, low molecular weight polypeptides, proteins, hydrophilic polymers, amino acids, sugars, chelating agents, counterions, metal complexes, and/or nonionic surfactants.

The pharmaceutically acceptable adjuvants may include buffers, antioxidants, preservatives, low molecular weight polypeptides, proteins, hydrophilic polymers, amino acids, sugars, chelating agents, counterions, metal complexes, and/or nonionic surfactants.

In the present application, the pharmaceutical composition can be formulated with a pharmaceutically acceptable carrier or diluent and any other known adjuvants and excipients according to conventional techniques in the art, for example, according to the technique disclosed in Remington: The Science and Practice of Pharmacy, 19th Edition, Edited by Gennaro, Mack Publishing Co., Easton, PA, 1995.

In the present application, the composition may be formulated for oral administration, intravenous administration, intramuscular administration, in situ administration at the tumor site, inhalation, rectal administration, vaginal administration, transdermal administration or administration via a subcutaneous depot.

In the present application, the composition can be used to inhibit tumor growth. For example, the compositions of the present application can inhibit or delay the development or progression of a disease (such as a tumor or an autoimmune disease), (for example, can reduce the size of the tumor, or even substantially eliminate the tumor), and/or can alleviate and/or stabilize the state of the disease.

The pharmaceutical composition described in the present application may comprise a therapeutically effective amount of the fusion protein. The therapeutically effective amount refers to the dose required to prevent and/or treat (at least in part) a disease (e.g., a neoplasm or an autoimmune disease) and/or any complications thereof in a subject suffering from or at risk of developing the disease.

In another aspect, the present application provides use of the SIRPα variant, fusion protein, nucleic acid molecule, vector, host cell and/or composition described in the present application in the preparation of drugs and/or kits, wherein the drugs and/or kits may be used for preventing or treating tumors or autoimmune diseases.

In the present application, the term "tumor" generally refers to a neoformation formed by the proliferation of local tissues and cells in the body, and because this neoformation is mostly a space-occupying lump, it is also called neoplasm. According to the cell characteristics and the extent of harm to the body of the neoformation, tumors are divided into two categories, benign tumors and malignant tumors. Cancer is the general term for malignant tumors. The tumors in the present application may include, but are not limited to, CD47-positive blood tumors or CD47-positive solid tumors.

In the present application, the term "CD47 positive blood tumor" generally refers to blood tumors that overexpress CD47, which may include various types of leukemia, lymphoma and myeloma. Said "leukemia" typically refers to a cancer of the blood in which too many white blood cell that do not function against infection are produced, thereby squeezing out other components, such as platelets and erythrocytes, that make up the blood. Leukemia can be divided into acute leukemia or chronic leukemia. Some forms of leukemia may be, for example, acute lymphocytic leukemia (ALL), acute myeloid leukemia (AML), chronic lymphocytic leukemia (CLL), chronic myeloid leukemia (CML), myeloproliferative disorders/neoplasms (MPDS), and myelodysplastic syndrome. The "lymphoma" may refer to Hodgkin's lymphoma, inert and aggressive non-Hodgkin's lymphoma, Burkitt's lymphoma, follicular lymphoma (small cell and large cell), etc. The myeloma may refer to multiple myeloma (MM), giant cell myeloma, heavy chain myeloma, light chain myeloma or Bence-Jones myeloma.

In the present application, the term "CD47-positive solid tumor" generally refers to solid tumors or concrete tumors that overexpress CD47, for which concrete lumps can be detected by clinical examination, for example, X-ray, CT scan, B-ultrasound, or palpation. Major categories may include carcinoma and sarcoma. For example, the CD47-positive solid tumor may include Ewing's sarcoma, osteosarcoma, rhabdomyosarcoma, bladder cancer, ovarian cancer, prostate cancer, lung cancer, colon cancer, breast cancer, pancreatic cancer, astrocytic carcinoma, glioblastoma, and renal cell carcinoma, etc.

In the present application, the autoimmune diseases may include Crohn's disease, allergic asthma, and rheumatoid arthritis.

In the present application, the term "Crohn's disease" generally refers to an inflammatory bowel disease of unknown cause, which can occur anywhere in the gastrointestinal tract but is more common in the terminal ileum and right colon. Both Crohn's disease and chronic non-specific ulcerative colitis are collectively known as inflammatory bowel disease (IBD).

In the present application, the term "allergic asthma" generally refers to chronic airway inflammation involving a variety of cells, particularly mast cells, eosinophils and T lymphocytes.

In the present application, the term "rheumatoid arthritis" generally refes to a chronic systemic autoimmune disease predominately with arthropathy.

The fusion protein, nucleic acid molecule, vector, host cell and/or composition described in the present application can be used to prevent or treat the tumor or the autoimmune disease.

In another aspect, the present application provides a method for preventing or treating tumors or autoimmune diseases, the method comprising administering the SIRPα variant, fusion protein, nucleic acid molecule, vector, host cell and/or composition described in the present application to a subject.

In another aspect, the present application provides a method for blocking the interaction between CD47 protein and SIRPα, which may include administering (e.g., administering to a subject in need thereof or cells or biological samples) the SIRPα variant, fusion protein or composition described in the present application.

In another aspect, the present application provides a method for inhibiting the growth and/or proliferation of tumors or tumor cells, which method may include bring the SIRPα variant, fusion protein or composition described in the present application into contact with the tumor or tumor cells. For example, the contact can occur in vitro.

In the present application, the term "subject" generally refers to any human or non-human animal. The term "non-human animal" may include all vertebrates, such as mammals and non-mammals, such as non-human primates, goats, sheep, dogs, cattle, chickens, amphibians, reptiles etc.

In the present application, the term "approximately" generally refers to a change in the range of 0.5% to 10% above or below a given value, for example, a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a given value.

In the present application, the term "including" generally means the meaning of comprising, covering, containing, or encompassing. In some cases, it also means "being" and "consisting of"

Without wishing to be bound by any theory, the following Examples are only for illustrating the SIRPα variant, fusion protein, preparation method and use of the present application, and are not used to limit the scope of the invention of the present application.

### EXAMPLES

### Example 1 Screening for variants

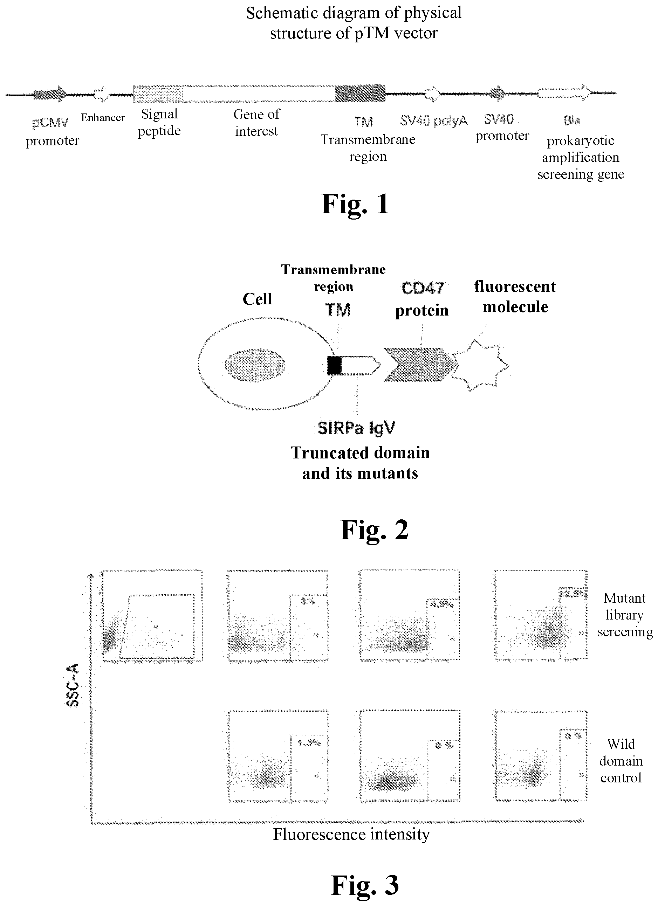

A truncated domain of the human SIRPα variant 1 (NP_542970.1) was obtained, the amino acid sequence of which is as set forth in SEQ ID NO: 2 (i.e., residues at position 33-149 in SEQ ID NO: 3). The structure of the truncated domain that interacts with human CD47 (CEJ95640.1) was constructed using Discovery Studio (NeoTrident Technology) software, the sites involved in the interaction in the two proteins and the interaction mode were theoretically analyzed, and it was determined that the amino acid sites that are directly or indirectly involved in the interaction with CD47 in the truncated domain are L44, I61, V63, E77, Q82, K83, E84, V93, D95, L96, K98, N100, R107, G109, N110, and V132 (wherein, the positions of the amino acid residues in these amino acid substitutions are numbered based on the amino acid sequence as set forth in SEQ ID NO: 3). Random mutation was performed on these action sites and a mutant library was constructed. This mutant library was then cloned into the vector pTM. The pTM vector contains a signal peptide and a transmembrane region sequence (as shown in Fig. 1), which can display the gene cloned into the vector on the cell surface.

The constructed mutant library expression vector was transfected into CHO cells (ATCC), so that the mutant library was displayed and expressed on the cell surface. Then, CD47 protein (Sino Biological) was fluorescently labeled with FITC to obtain CD47-FITC. Based on the binding activity between CD47-FITC and mutants of the truncated domain on the surface of CHO cells, flow cytometry was used to enrich and screen the mutants that could bind to CD47-FITC. The specific principle of screening can be seen in Fig. 2, in which the truncated domain and the mutant bind to the CD47 protein with the fluorescent molecule, and the result of the binding can be reflected by the level of the fluorescent molecule.

After four rounds of screening and enrichment, cells with strong binding to CD47-FITC were collected (as shown in Fig. 3). Then its mRNA was extracted, cDNA was obtained after reverse transcription, and the gene of the truncated domain mutant was sequenced and analyzed. The sequencing results show that different mutation combinations existed at the aforementioned sites L44, 161, V63, E77, Q82, K83, E84, V93, D95, L96, K98, N100, R107, G109, N110, and V132.

The results show that by introducing different mutation combinations at residues L44, I61, V63, E77, Q82, K83, E84, V93, D95, L96, K98, N100, R107, G109, N110 and/or V132, new truncated domain mutants that can specifically recognize CD47 can be obtained.

Further analysis of the mutation sites revealed that the amino acid residues mutated at the respective sites are: L44V, I61L/V/F, V63I, E77I/N/Q/K/H/M/R/N/V/L, Q82S/R /G/N, K83R, E84Q/K/H/D/R/G, V93L/A, D95H/R/E, L96S/T, K98R, N100G/K/D/E, R107N/S, G109R/H, N110A, and V132L/R/I/S.

Based on the above site analysis, variants of the SIRPα domain with the following amino acid mutations were obtained:
(1) I61L, V63I, E77I, E84K, V93L, L96S, K98R, N100G, N110A and V132L (SEQ ID NO: 8, m1N);
(2) I61V, E77N, Q82S, K83R, E84H and N110A (SEQ ID NO: 9, m5N);
(3) I61F, V63I, K83R, E84K, N110A and V132I (SEQ ID NO: 10, m170N);
(4) I61L, E77Q, E84D, R107N, N110A and V132I (SEQ ID NO: 11, m35N);
(5) I61L, V63I, E77K, K83R, E84D, N100G and N110A (SEQ ID NO: 12, m37N);
(6) I61V, E77H, Q82R, K83R, E84H, R107S and N110A (SEQ ID NO: 13, m41N);
(7) I61L, E77I, Q82G, E84R, V93L, L96T, N100G, R107S, G109R, N110A and V132R (SEQ ID NO: 14, m57N);
(8) 161L, E77M, Q82G, K83R, E84D, N110A and V132L (SEQ ID NO: 15, m67N);
(9) I61L and N110A (SEQ ID NO: 16, m81N);
(10) I61F, D95H, L96S, G109H, N110A and V132S (SEQ ID NO: 17, m82N);
(11) I61F, D95H, L96S, K98R, G109H, N110A and V132S (SEQ ID NO: 18, m84N);
(12) I61L, E77Q, E84D, V93A, R107N, N110A and V132I (SEQ ID NO: 19, m91N);
(13) E77K, L96S, N100K, G109H, N110A and V132L (SEQ ID NO: 20, m99N);
(14) I61L, V63I, Q82G, E84G, D95R, L96S, N100D, N110A and V132I (SEQ ID NO: 21, m102N);
(15) I61L, E77R, Q82N, K83R, E84G, V93L, D95E, L96T, K98R, N100D, N110A, and V132L (SEQ ID NO: 22, m111N);
(16) 161V, E77N, Q82S, K83R, E84H, V93A and N110A (SEQ ID NO: 23, m122N);
(17) 161V, V63I, E77V, K83R, E84D, D95E, L96T, K98R, N100E and N110A (SEQ ID NO: 24, m126N);
(18) I61L, V63I, E77V, K83R, D95E, L96S, K98R, N100D, G109R and N110A (SEQ ID NO: 25, m130N);
(19) 161V, E77L, Q82G, E84G, V93L, D95E, L96T, K98R, N100G and N110A (SEQ ID NO: 26, m135N);
(20) I61L, V63I, E77N, Q82G, E84G and N110A (SEQ ID NO: 27, m145N); and
(21) L44V, I61F, E77I, Q82R, K83R, E84Q, N110A and V132I (SEQ ID NO: 1, m12N).

The truncated domain of the human SIRPα variant 1 (or called the wild-type SIRPα truncated domain, the sequence of which is as set forth in SEQ ID NO: 2) was subjected to N110A amino acid mutation, and the variant of the SIRPα domain obtained in Example 1 (the amino acid sequence of which is as set forth in SEQ ID NOs: 1, and 8-27, respectively) was fused with human IgG1-Fc (the amino acid sequence of which is as set forth in SEQ ID NO: 4) to obtain the corresponding truncated domain-human Fc fusion protein of SIRPα mutant 1 (referred to as fusion protein for short), wherein the fusion protein of wild-type SIRPα truncated domain and Fc was named SS002, the fusion protein of wild-type SIRPα truncated domain (containing N110A mutation) and Fc was named SS002N, and other SIRPα variant-IgG1-Fc fusion proteins were named SS002m1N, SS002m5N, SS002m170N, SS002m35N, SS002m37N, SS002m41N, SS002m57N, SS002m67N, SS002m81N, SS002m82N, SS002m84N, SS002m91N, SS002m99N, SS002m102N, SS002m111N, SS002m122N, SS002m126N, SS002m130N, SS002m135N, SS002m145N and SS002m12N respectively.

### Example 2 Detection of the effect of binding of fusion protein to target antigen CD47

The target antigen His-CD47 (CD47 Protein, Human, Recombinant (ECD, His Tag), SinoBiological) was coated on the ELISA strip plate, at 1 µg/ml, and coated overnight at 4°C; after washing with PBST, 10% fetal bovine serum was added to block at 37°C for 1 h; fusion proteins of different concentrations were added and the system was allowed to react at 37°C for 1 h; after washing with PBST, horseradish peroxidase-labeled goat anti-human Fc secondary antibody (Goat anti-Human IgG Fc Cross-Adsorbed Secondary Antibody, HRP, Invitrogen) was added, the system was allowed to react at 37°C for 30 min; the plate was washed with PBST repeatedly for 5 times, and the remaining droplets was pat dry on the absorbent paper as far as possible; 100 µl TMB (eBioscience) was added to each well, and the plate was placed at room temperature (20±5°C) in the dark for 2-3 min; 100 µl 2N H₂SO₄ stop solution was added to each well to terminate the substrate reaction, the OD value was read at 450 nm with a microplate reader, and the ability of the fusion protein to bind to CD47 was analyzed.

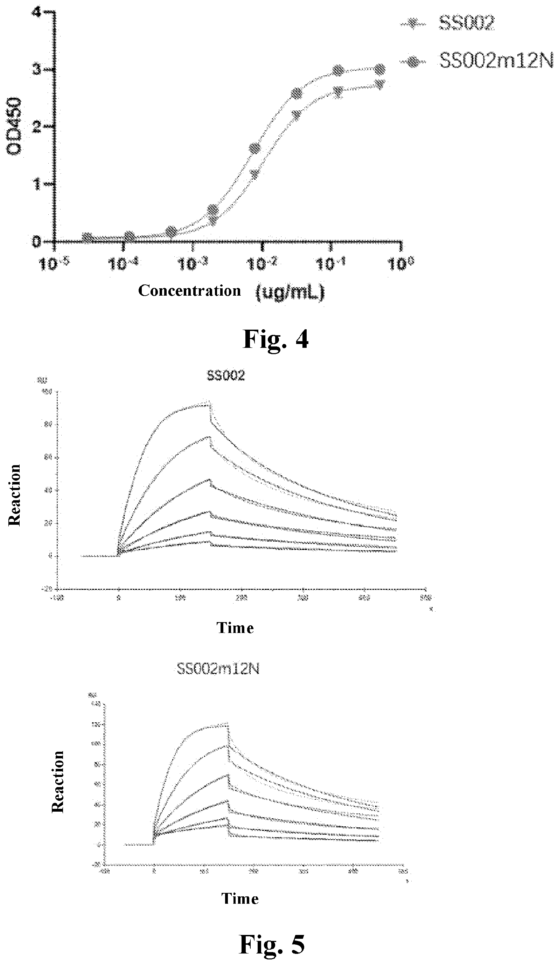

The results are as shown in Fig. 4. The fusion protein can specifically recognize the target antigen His-CD47, and the binding activity is significantly dose-dependent; compared with SS002N, SS002m12N has a stronger binding ability to the target antigen His-CD47.

### Example 3 Detection of affinity between fusion protein and target antigen CD47

The affinity between the fusion protein and His-CD47 was detected by using surface plasmon resonance (SPR) technology. The instrument used in the experiment was Biacore T200 (GE). According to the instructions for Human Antibody Capture Kit (GE), Anti-Human IgG (Fc) antibody (including in GE Human Antibody Capture Kit, GE) was diluted with Immobilization Buffer (including in GE Human Antibody Capture Kit, GE) to 25 µg/ml and fixed on Series S Sensor Chip CM5 chip (GE) (10 µl/min, 420s); the fusion protein was diluted to 2.5 µg/ml with Running Buffer (1×HEPES (10 mM HEPES, 150 mM NaCl, 3 mM EDTA), with 0.005 %Tween-20, pH7.4) and captured at a flow rate of 10 µl/min; and His-CD47 (CD47 Protein, Human, Recombinant (ECD, His Tag), SinoBiological) was used as the analyte for association and dissociation test (30 µl/min, Association: 150 s; Dissociation 300 s), and fitted with a 1:1 binding model to analyze the affinity between the fusion protein and CD47.

The results are shown in Table 1 and Fig. 5. All the fusion proteins can bind to CD47 molecules with high affinity; SS002m12N, SS002m91N, SS002m82N, and SS002m84N have stronger affinity with CD47 molecules compared with SS002N.

**Table 1 Detection results of affinity between fusion protein and His-CD47**

| **Ligand** | | **1:1 binding** | | |
|---|---|---|---|---|
| | **capture level (RU)** | **ka (1/Ms)** | **kd (1/s)** | **KD (M)** |
| **SS002m91N** | 307 | 1.96E+05 | 8.29E-04 | **4.23E-09** |
| **SS002** | 310 | 3.27E+05 | 6.39E-03 | **1.95E-08** |
| **SS002m5N** | 337 | 3.24E+05 | 6.46E-03 | **1.99E-08** |
| **SS002m12N** | 340 | 3.33E+05 | 4.45E-03 | **1.34E-08** |
| **SS002m82N** | 347 | 2.57E+05 | 2.78E-03 | **1.08E-08** |
| **SS002m84N** | 328 | 2.27E+05 | 2.09E-03 | **9.18E-09** |

### Example 4 Fusion protein blocks the binding of CD47 to SIRPα

His-SIRPα (SIRP alpha Protein, Human, Recombinant (G75A, ECD, His Tag), SinoBiological) was coated on the ELISA strip plate, at 1 µg/ml, and coated overnight at 4°C; after washing with PBST, 10% fetal bovine serum was added to block at 37°C for 1 h; fusion proteins of different concentrations and 2 µg/ml CD47-Biotin (Jiaxuan Biotech) were added and the system was allowed to react at 37°C for 1 h; after washing with PBST, horseradish peroxidase labeled-avidin (Streptavidin, HRP conjugate, Jiaxuan Biotech) was added and the system was allowed to react at 37°C for 30 min; the plate was washed with PBST repeatedly for 5 times, and the remaining droplets was pat dry on absorbent paper as far as possible; 100 µl TMB (eBioscience) was added to each well, and the plate was placed at room temperature (20±5°C) in the dark for 2-3 min; 100 µl 2N H₂SO₄ stop solution was added to each well to terminate the substrate reaction, the OD value was read at 450 nm with a microplate reader, and the ability of the fusion protein to block the binding of CD47 to SIRPα was analyzed.

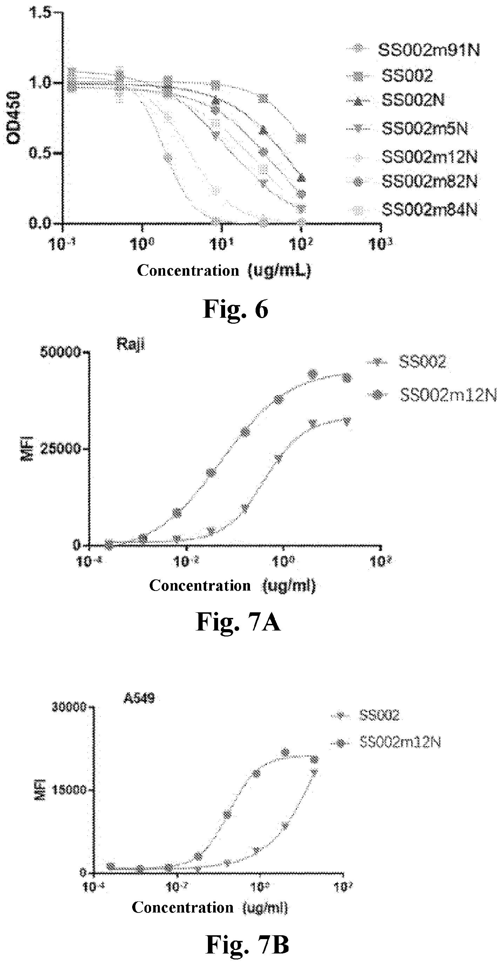

The results are shown in Fig. 6. The fusion protein can block the binding of CD47 to SIRPα, and the blocking activity is significantly dose-dependent; and the blocking activity of SS002m91N, SS002m5N, SS002m12N, SS002m82N, and SS002m84N described in the present application is stronger than that of SS002 and SS002N.

### Example 5 Fusion protein specifically recognizes CD47 molecules on the surface of tumor cells

The activity of the fusion protein to bind to CD47 on the surface of Raji cells and A549 cells was detected by using flow cytometry (BD Accuri C6 Plus), respectively. Raji and A549 cells at logarithmic growth phase were collected and added to 1.5 ml EP tubes at 5×10⁵ cells per tube, respectively; different concentrations of fusion proteins were added, respectively and the system was incubated on ice in the dark for 30 min; after washing with FACS wash buffer, PE fluorescently labeled goat anti-Human IgG Fc secondary antibody (Goat Anti-Human IgG Fc Secondary Antibody , PE , Invitrogen) was added and the system was incubated on ice in the dark for 30 min; those were washed twice with FACS wash buffer; 400 µl of 1% paraformaldehyde fixative (Solarbio) was added to each tube to fix the cells. The system was well mixed and detected for the relative fluorescence intensity of PE fluorescence on the machine to analyze the ability of the fusion protein to bind to CD47 on the surface of Raji cells and A549 cells.

The results are shown in Figs. 7A and 7B. The fusion protein can specifically recognize CD47 on the surface of Raji cells and A549 cells, and the binding activity is significantly dose-dependent; and compared with SS002, SS002m12N has a stronger ability to bind to tumor cells.

The fusion proteins of other variants constructed in Example 1 were further selected, and the ability of the fusion proteins to bind to CD47 on the surface of Raji cells and A549 cells was analyzed again, using SS002N as a control.

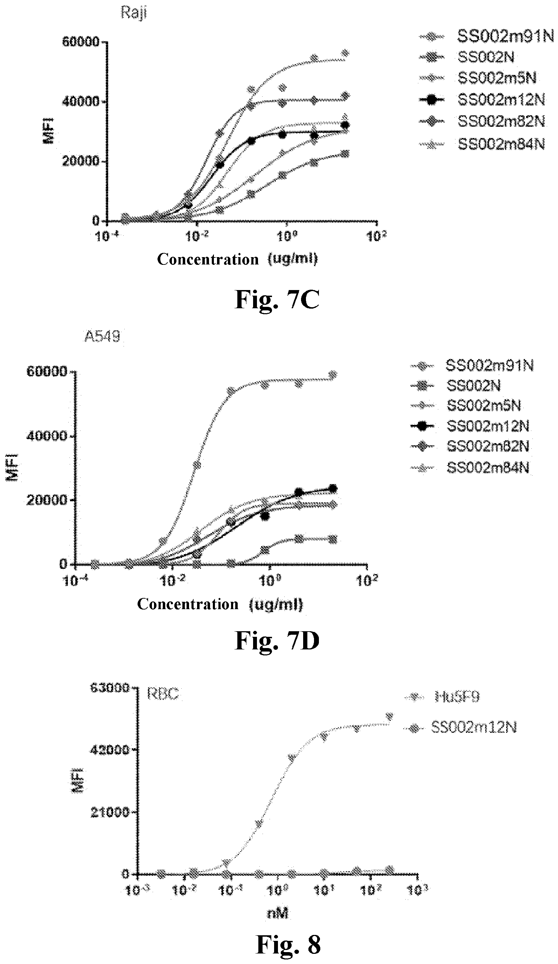

The results are shown in Figs. 7C and 7D, which show that SS002m12N, SS002m5N, SS002m82N, SS002m84N, and SS002m91N all show stronger tumor cell binding ability compared with SS002N.

### Example 6 Binding of fusion protein to human erythrocytes

The binding of the fusion protein to human erythrocytes was detected by using flow cytometry (BD Accuri C6 Plus), using CD47 antibody Hu5F9-G4 as a control (see Guerriero JL, Sotayo A, Ponichtera HE, et al. Class IIa HDAC inhibition reduces breast tumors and metastases through anti-tumour macrophages. [J]. Nature, 2017, 543(7645): 428.432 and Gholamin S, Mitra SS, Feroze AH et al. Disrupting the CD47-SIRPα anti-phagocytic axis by a humanized anti-CD47 antibody is an efficacious treatment for malignant pediatric brain tumors. Sci. Transl. Med 2017). Whole blood from healthy donors (peripheral blood collected from volunteers) was collected to prepare human erythrocytes, diluted 10-fold with physiological saline (Shijiazhuang No.4 Pharmaceutical), washed three times, and prepared into a fresh 1% (v/v) erythrocyte suspension. The cells were added to 1.5 ml EP tubes at 1×10⁶ cells per tube, and washed twice with FACS wash buffer (4°C, 3000 rpm, 5 min); 100 ul of fusion proteins at different concentrations were added and the system was incubated on ice in the dark for 30 min, and mixed once every 10 min; after washing with FACS wash buffer, PE fluorescently labeled goat anti-human IgG Fc secondary antibody (Goat Anti-Human IgG Fc Secondary Antibody , PE , Invitrogen) was added, and the system was incubated on ice in the dark for 30 min; those were washed twice with FACS wash buffer; 400 µl of 1% paraformaldehyde fixative (Solarbio) was added to each tube to fix the cells, and the system was mixed well and detected for the relative fluorescence intensity of PE fluorescence on the machine to analyze the ability of the fusion protein to bind to human erythrocytes.

The results are shown in Fig 8. SS002m12N hardly binds to human erythrocytes, whereas Hu5F9-G4 strongly binds to human erythrocytes, and its binding activity is significantly dose-dependent.

### Example 7 Hemagglutination test of fusion protein

Whole blood from healthy donors (peripheral blood collected from volunteers) was collected to prepare human erythrocytes, diluted 10-fold with physiological saline (Shijiazhuang No.4 Pharmaceutical), washed three times, and prepared into a fresh 1% (v/v) erythrocyte suspension. A 96-well Round Bottom Cell Culture plate (96-well Clear Round Bottom TC-treated Microplate, Corning) was taken, and different concentrations of fusion protein (50 ul/well) and the 1% erythrocyte suspension (50 ul/well) were added, and mixed thoroughly. The system was put in a wet box, incubated at 37 °C for 3.5 h, and then observed for the agglutination phenomenon.

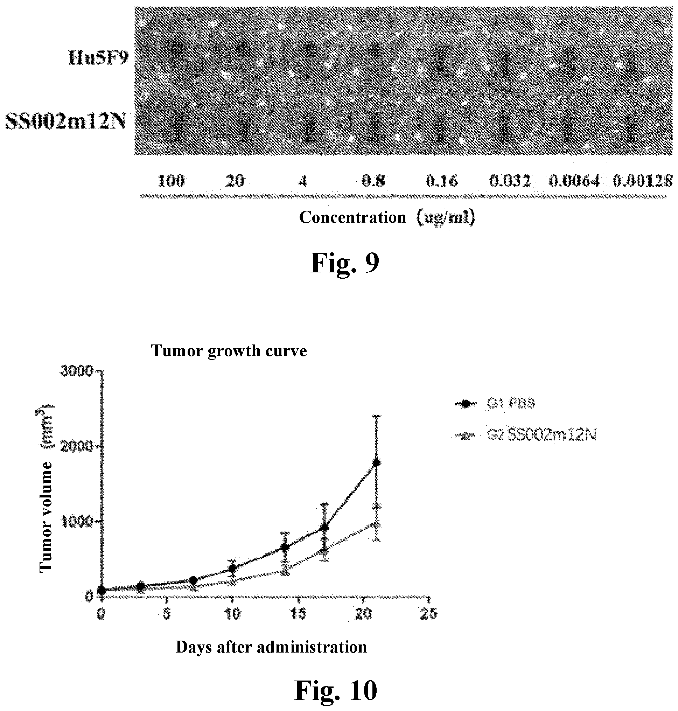

The results are shown in Fig. 9. SS002m12N has no hemagglutination in the concentration range of 0.00128 µg/ml-100 µg/ml; while Hu5F9-G4 can cause hemagglutination in the concentration range of 0.8 µg/ml-100 µg/ml.

### Example 8 Detection of tumor inhibitory activity of fusion protein in vivo

A tumor model was established by xenografting CD47-positive human cutaneous squamous cell carcinoma cells A431 in NOD-SCID mice to evaluate the tumor inhibitory activity of the fusion protein. Female, 6-8-week old NOD-SCID mice (InnoModels Biotechnology (Beijing) Co., Ltd.) were selected as experimental animals. After resuscitated and cultured to the required number, the A431 cells at the logarithmic growth phase were collected and suspended to a concentration of 1×10⁶ cells/0.1mL. Then, the cells were subcutaneously inoculated at 0.1 mL/mouse into NOD-SCID mice at the scapula of the right forelimb. Tumor volume and body weight were measured twice a week after inoculation. When the average tumor volume reached 99 mm³, the mice were randomized into 2 groups based on tumor volume and body weight, with 6 animals in each group, including a solvent control group (G1, PBS) and an experimental group (G2, SS002m12N). The administrated dose of the experimental group was 7 mg/kg, and administration was carried out continuously at a frequency of twice a week on the day of randomization and after the randomization, for a total of 6 times. Tumor growth in mice was observed.

The results are shown in Fig 10. On the 21st day after group administration, the average tumor volume of the control group was 1795.5±611.6mm³, while the average tumor volume of the administration group was 994.2±239.22mm³, with a tumor inhibition rate of 47%, showing a tumor inhibition effect.

## Claims

1. An SIRPα variant, comprising an amino acid substitution at the amino acid residue ofN110, compared with a domain of the human SIRPα variant 1 or a fragment thereof.

2. The SIRPα variant according to claim 1, wherein the domain of the human SIRPα variant 1 or fragment thereof comprises the amino acid residues at position 33-149 of the human SIRPα variant 1.

3. The SIRPα variant according to any one of claims 1-2, wherein the amino acid residues at position 33-149 of the human SIRPα variant 1 comprise the amino acid sequence as set forth in SEQ ID NO: 2.

4. The SIRPα variant according to any one of claims 1-3, comprising amino acid substitution N110A.

5. The SIRPα variant according to any one of claims 1-4, further comprising an amino acid substitution at one or more residues selected from the group consisting of: L44, I61, V63, E77, Q82, K83, E84, V93, D95, L96, K98, N100, R107, G109 and V132.

6. The SIRPα variant according to any one of claims 1-5, further comprising one or more amino acid substitutions selected from the group consisting of: L44V, I61L/V/F, V63I, E77I/N/Q/K/H/M/R/V/L, Q82S/R/G/N, K83R, E84Q/K/H/D/R/G, V93L/A, D95H/R/E, L96S/T, K98R, N100G/K/D/E, R107N/S, G109R/H and V132L/R/I/S.

7. The SIRPα variant according to any one of claims 1-6, comprising substitutions at amino acid residues selected from any of the following groups:
(1) I61, V63, E77, E84, V93, L96, K98, N100, N110 and V132;
(2) 161, E77, Q82, K83, E84 and N110;
(3) 161, V63, K83, E84, N110 and V132;
(4) 161, E77, E84, R107, N110 and V132;
(5) 161, V63, E77, K83, E84, N100 and N110;
(6) 161, E77, Q82, K83, E84, R107 and N110;
(7) 161, E77, Q82, E84, V93, L96, N100, R107, G109, N110 and V132;
(8) 161, E77, Q82, K83, E84, N110 and V132;
(9) I61 and N110;
(10) 161, D95, L96, G109, N110 and V132;
(11) I61, D95, L96, K98, G109, N110 and V132;
(12) 161, E77, E84, V93, R107, N110 and V132;
(13) E77, L96, N100, G109, N110 and V132;
(14) 161, V63, Q82, E84, D95, L96, N100, N110 and V132;
(15) 161, E77, Q82, K83, E84, V93, D95, L96, K98, N100, N110 and V132;
(16) 161, E77, Q82, K83, E84, V93 and N110;
(17) 161, V63, E77, K83, E84, D95, L96, K98, N100 and N110;
(18) 161, V63, E77, K83, D95, L96, K98, N100, G109 and N110;
(19) 161, E77, Q82, E84, V93, D95, L96, K98, N100 and N110;
(20) 161, V63, E77, Q82, E84 and N110; and
(21) L44, 161, E77, Q82, K83, E84, N110 and V132.

8. The SIRPα variant according to any one of claims 1-7, comprising amino acid mutations selected from any of the following groups:
(1) I61L, V63I, E77I, E84K, V93L, L96S, K98R, N100G, N110A and V132L;
(2) 161V, E77N, Q82S, K83R, E84H and N110A;
(3) I61F, V63I, K83R, E84K, N110A and V132I;
(4) I61L, E77Q, E84D, R107N, N110A and V132I;
(5) I61L, V63I, E77K, K83R, E84D, N100G and N110A;
(6) I61V, E77H, Q82R, K83R, E84H, R107S and N110A;
(7) I61L, E77I, Q82G, E84R, V93L, L96T, N100G, R107S, G109R, N110A and V132R;
(8) I61L, E77M, Q82G, K83R, E84D, N110A and V132L;
(9) I61L and N110A;
(10) I61F, D95H, L96S, G109H, N110A and V132S;
(11) I61F, D95H, L96S, K98R, G109H, N110A and V132S;
(12) I61L, E77Q, E84D, V93A, R107N, N110A and V132I;
(13) E77K, L96S, N100K, G109H, N110A and V132L;
(14) I61L, V63I, Q82G, E84G, D95R, L96S, N100D, N110A and V132I;
(15) I61L, E77R, Q82N, K83R, E84G, V93L, D95E, L96T, K98R, N100D, N110A, and V132L;
(16) I61V, E77N, Q82S, K83R, E84H, V93A and N110A;
(17) 161V, V63I, E77V, K83R, E84D, D95E, L96T, K98R, N100E and N110A;
(18) I61L, V63I, E77V, K83R, D95E, L96S, K98R, N100D, G109R and N110A;
(19) 161V, E77L, Q82G, E84G, V93L, D95E, L96T, K98R, N100G and N110A;
(20) I61L, V63I, E77N, Q82G, E84G and N110A; and
(21) L44V, 161F, E77I, Q82R, K83R, E84Q, N110A and V132I.

9. The SIRPα variant according to any one of claims 1-8, comprising the amino acid sequence as set forth in any one of SEQ ID NOs: 1, and 8-27.

10. A fusion protein, comprising the SIRPα variant of any one of claims 1-9, and an immunoglobulin Fc region.

11. The fusion protein according to claim 10, wherein the immunoglobulin Fc region comprises an IgG Fc region or a variant thereof.

12. The fusion protein according to claim 11, wherein the IgG is selected from the group consisting of IgG1, IgG2, IgG3 and IgG4.

13. The fusion protein according to any one of claims 10-12, wherein the SIRPα variant is located at the N-terminus of the immunoglobulin Fc region.

14. The fusion protein according to any one of claims 10-13, wherein the immunoglobulin Fc region comprises an amino acid sequence selected from the group consisting of SEQ ID NOs: 4-5.

15. The fusion protein according to any one of claims 10-14, comprising the amino acid sequence as set forth in any one of SEQ ID NOs: 6-7.

16. The fusion protein according to any one of claims 10-15, specifically binding to CD47 protein and having at least one of the following properties:
1) binding to CD47 protein with a *K_{D}* value of 1×10⁻⁸M or lower;
2) specifically blocking the interaction between CD47 protein and SIRPα;
3) not inducing coagulation reaction; and
4) inhibiting the growth and/or proliferation of tumors or tumor cells.

17. The fusion protein according to claim 16, wherein the CD47 protein is human CD47 protein.

18. One or more isolated nucleic acid molecules, encoding the SIRPα variant of any one of claims 1-9, or the fusion protein of any one of claims 10-17.

19. A vector, comprising the nucleic acid molecule of claim 18.

20. A host cell, comprising the nucleic acid molecule of claim 18 or the vector of claim 19.

21. A method for preparing the SIRPα variant of any one of claims 1-9 or the fusion protein of any one of claims 10-17, comprising culturing the host cell of claim 20 under conditions that allow the expression of the SIRPα variant or the fusion protein.

22. A composition, comprising the SIRPα variant of any one of claims 1-9, the fusion protein of any one of claims 10-17, the nucleic acid molecule of claim 18, the vector of claim 19 and/or the host cell of claim 20, and optionally a pharmaceutically acceptable adjuvant.

23. Use of the SIRPα variant of any one of claims 1-9, the fusion protein of any one of claims 10-17, the nucleic acid molecule of claim 18, the vector of claim 19, the host cell of claim 20 and/or the composition of claim 22 in the preparation of drugs and/or kits for preventing or treating tumors or autoimmune diseases.

24. The use according to claim 23, wherein the tumor is selected from the group consisting of CD47 positive hematological tumors or CD47 positive solid tumors.

25. The use according to claim 23, wherein the autoimmune disease is selected from the group consisting of Crohn's disease, allergic asthma and rheumatoid arthritis.

26. A method for blocking the interaction between CD47 protein and SIRPα, comprising administering the fusion protein of any one of claims 10-17 or the composition of claim 22.
